# EUROPEAN PATENT APPLICATION

(11) **EP 4 574 156 A1**
(43) Date of publication of application: **25.06.2025**
(21) Application number: 23219929.9
(22) Date of filing: 22.12.2023
(51) Int. Cl.: A61K 31/732, B01D 15/18, B01D 15/36, C07H 1/06, C08B 37/00

(54) **REFINEMENT OF URONIC ACID CONTAINING HYDROLYSATES**

(71) Applicant: Coöperatie Koninklijke Cosun U.A., 4814 NE Breda (NL)
(72) Inventor: VAN DEN BERGH, Johan, 4814 NE Breda (NL); HUIJGEN, Wouter Johannes Joseph, 4814 NE Breda (NL)
(74) Representative: Nederlandsch Octrooibureau

(57) **Abstract**

The present invention relates to methods of enriching uronic acid containing compositions using anion-exchange chromatography and to the compositions obtainable by said methods.

## Description

### Field of the Invention

The present invention relates to methods of processing uronic acid containing compositions, especially to methods of enriching the uronic acid content of such compositions. These methods are particularly suitable in the (industrial-scale) production of high-purity uronic acid from biomass such as hydrolysates of pectin or more complex biomass hydrolysate such as plant material hydrolysate, algae hydrolysate or bacteria hydrolysate. The present invention also pertains to the enriched uronic acid and monosaccharide compositions that can be obtained with these methods and have certain beneficial properties.

### Background of the Invention

Galacturonic acid is one of the main building blocks of pectin, a material found in raw plant materials and various agro-food by-products, such as citrus (peels), apple (pommace), (sweet) potato (peels), carrot (pomace), (spent) coffee (grounds), sugar beet (pulp), and chicory root (pulp). Sugar beet pulp and chicory root pulp, in particular, are rich sources of pectin and thus galacturonic acid. Hydrolysates of pectin consist mainly of L-arabinose and D-galacturonic acid, but includes in lower quantities, other monosaccharides such as glucose, other uronic acids, (pectic) oligosaccharides, organic acids such as acetic acid as well as salts and methanol.

As the transition from a fossil-based to a bio-based economy is starting to take shape, galacturonic acid and other uronic acids are attracting attention as a bio-based chemical building block. For example, oxidation of galacturonic acid efficiently yields the corresponding aldaric acid, galactaric or mucic acid. Galactaric acid can for example be applied as chelating agent in cosmetics and as anticorrosion agent. Galactaric acid can e.g. be converted into 2,5- furandicarboxylic acid (FDCA), a widely advocated bio-based alternative for terephthalic acid, used in polyesters like PET. Alternatively, galactaric acid can be converted into a family of derivatives, conventionally called GaIX, which have been shown to have interesting properties when incorporated into conventional polyesters. Applications of uronic acids and/or building blocks based thereon are currently envisaged in numerous fields of industry, including food, pharma, coatings, composites, flavours, cosmetics, detergents, chemicals, animal feed, etc.

Society nowadays demands that feedstocks used in industry are renewable, bio-based and do not compete with food production. Hence, agricultural residues such as sugar beet pulp (SBP), citrus pulp, citrus peel, chicory root pulp, etc. would be the ideal source for the recovery of galacturonic acid for the production of bio-based chemical building blocks.

Despite its promises, the use of uronic acids like galacturonic acid has been hampered, primarily by the absence of techniques that allow for recovery of uronic acids like galacturonic acid at costs sufficiently low to permit economical large-scale operations. Galacturonic acid can be obtained by hydrolysis of isolated pectin or by hydrolysis of (whole) plant, bacterial or algae material. These processes are often based on the enzymatic hydrolysis and liquefication of residual root crops such as sugar beet pulp and chicory root pulp. Enzymatic digestion by a wide range of enzymes capable of hydrolyzing the carbohydrate cell wall components including pectin yields a hydrolysate containing galacturonic acid in admixture with other components like organic acids, sugars, residual pectic oligomers (which can be measured as galacturonic acid oligomers described herein in more detail later on), etc. A bottleneck for exploiting galacturonic acid to its full potential, is the lack of availability of techniques for purification of galacturonic acid from the hydrolysate that can be implemented on a large scale, as part of an integrated biorefinery concept, in an economically feasible manner.

The separation of sugars and sugar acids such as uronic acids from each other and/or their oligomers is traditionally a difficult process that is chemically intensive and costly. To date, only a limited amount of work has been reported on the purification of galacturonic acid from pectin hydrolysates and comparable matrices. Very old literature describes purification methods based on the formation of certain salts of galacturonic acid that have relatively low solubility in water and crystallizes, from a pectin hydrolysate, in high purity. This technique is described, for instance in US 2,338,534, which focuses on the formation of the potassium calcium double salt of galacturonic acid. Even though high purity yields are obtainable by such processes, it is chemically intensive, costly and incompatible with modern-day sustainability standards.

As an alternative option, galacturonic acid can be separated from other neutral sugars in pectin hydrolysates using ion exchange techniques, wherein an anion exchange resin is used to bind galacturonic acid, followed by elution using a regenerating salt. For example, Suzuki et al. (Preparation and isolation of oligogalacturonic acids and their biological effects in cockscomb (Celosia argentea L.) seedlings, J. Plant Growth Regul. 21 (2002) 209-215) separated a mixture of oligogalacturonic acids on a DEAE Sephadex A-25 resin, which is an anion exchanger, using NH₄HCO₃ as the eluent.

To date, ion exchange is probably the most effective technology to provide enriched galacturonic acid containing compositions from a pectin hydrolysate, but it has significant disadvantages and is mostly used due to a lack of alternatives. For example, large amounts of salts are usually required, while yields are still modest due to significant degradation of galacturonic acid. All things considered, ion exchange is too costly and not a sustainable method for large scale purification of galacturonic acid.

It is an object of the present invention to provide a method of enriching a crude uronic acid composition that is economical and provides high yields of purified uronic acid.

### Summary of the Invention

To this end, the present invention provides a method of enriching a uronic acid composition by use of a chromatographic separation process with a stationary phase comprising an anion exchange resin. The method according to the invention provides an enriched uronic acid composition in an economically feasible manner and allows very high purities to be achieved. It has been found that the method of the present invention allows a surprisingly efficient separation of uronic acids such as galacturonic acid from monosaccharides and oligomeric forms of the uronic acid, such that uronic acid compositions having a very high uronic acid content can be obtained. The efficient separation between the uronic acid and oligomeric forms of the uronic acid is particularly surprising since uronic acid polymers (such as pectin) retain a large amount of free (non-esterified) acid groups and would thus be expected to behave similar to galacturonic acid in a basic resin chromatographic separation. An additional advantage of the present method is that uronic acids can be recovered in their free acid form, thereby avoiding the need for conversion of the uronic acid salt into its free acid form which arises when for example ion-exchange purification methods are used.

The inventors believe that the method according to the invention, is an improvement over the methods taught by the prior art, especially from the process economics and environmental perspectives, and, further, that it provides enriched uronic acid and monosaccharide compositions that have certain advantageous product characteristics over those obtained with the prior art methods, as will be apparent from the detailed description and experimental results presented herein.

Hence, a first aspect of the invention concerns a method of enriching a uronic acid containing composition, said method comprising the consecutive steps of:
a) providing a crude uronic acid composition comprising a first uronic acid and one or more monosaccharides;
b) subjecting the crude uronic acid composition of step a) to a chromatographic separation using a stationary phase comprising an anion exchange resin;
c) collecting an enriched uronic acid composition.

In further aspects, the invention pertains to the intermediate and end-products obtainable by the methods described herein.

These and other aspects of the invention, and preferred embodiments thereof, will be described and illustrated in more detail in the following sections.

In all embodiments of the invention, the first uronic acid is preferably selected from the group consisting of galacturonic acid, mannuronic acid, guluronic acid, glucuronic acid, iduronic acid, and combinations thereof, preferably the uronic acid is galacturonic acid.

### Brief description of the figures

Figure 1 shows the results of example 1 wherein the chromatographic separation was performed with Amberlite^{™} CR5550.
Figure 2 shows the results of example 1 wherein the chromatographic separation was performed with and Amberlite^{™} CR7300.

### Detailed Description of the Invention

The expression "comprise" and variations thereof, such as, "comprises" and "comprising" as used herein should be construed in an open, inclusive sense, meaning that the embodiment described includes the recited features, but that it does not exclude the presence of other features, as long as they do not render the embodiment unworkable. The word 'distinct' highlights that the distinct steps of the process steps are different.

The expressions "one embodiment", "a particular embodiment", "an embodiment" etc. as used herein should be construed to mean that a particular feature, structure or characteristic described in connection with the embodiment is included in at least one embodiment. Thus, the appearances of such expressions in various places throughout this specification do not necessarily all refer to the same embodiment. Furthermore, the particular features, structures, or characteristics may be combined in any suitable manner in one or more embodiments. For example, certain features of the disclosure which are described herein in the context of separate embodiments are also explicitly envisaged in combination in a single embodiment.

The singular forms "a," "an," and "the" as used herein should be construed to include plural referents unless the content clearly dictates otherwise. It should also be noted that the term "or" is generally employed in its broadest sense, that is as meaning "and/or" unless the content clearly dictates otherwise.

The term "biomass hydrolysate" denotes biomass that has been subjected to processing to decrease the average molecular weight of one or more polysaccharides comprised in the biomass. Such biomass hydrolysates are typically obtained by chemical (acid or base), hydrothermal and/or enzymatic digestion. Preferably in the context of the present invention the biomass hydrolysate denotes biomass that has been subjected to processing to decrease the average molecular weight of one or more polysaccharides comprised in the biomass by hydrolytic cleavage. It is preferred that at least 70 mol% of the uronic acid in polymeric form which is present in the biomass before hydrolysis is present in the hydrolysate in monomeric form, i.e. as uronic acid. The total combined amount of uronic acid in monomeric and polymeric form present in the biomass before hydrolysis can routinely be determined by the skilled person for each uronic acid using a chemical (e.g. Seaman) post-hydrolysis, or by enzymatic post-hydrolysis using high dose of enzymes such that a complete hydrolysis is achieved, followed by determination of the content of various sugars and uronic acids by e.g. chromatographic analysis as described herein elsewhere, or by a colorimetric method as described in M. Blumenkranz and G Asboe-Hansen New method for the determination of uronic acids Anal. Biochem. 54, 484 (1973) or R.R. Selvendran, J.F. March and S.G. Ring Determination of aldoses and uronic acid content of vegetable fibre Anal.Biochem. 96, 282 (1979). Enzymatic post-hydrolysis using high dose of enzymes such that a complete hydrolysis is achieved, followed by determination of the content of various sugars and uronic acids by chromatographic analysis as described herein elsewhere is preferred.

The term "pectin" as used herein refers to a class of plant cell-wall heterogeneous polysaccharides predominantly containing galacturonic acid. Pectin can be extracted from plant material by treatment with acids, chelating agents, using hot water or specific solvents. Typically, 70-80% of pectin is found as a linear chain of o(1-4)-linked D-galacturonic acid monomers (homogalacturonan).

The term "hemicellulose" refers to a class of plant cell-wall polysaccharides that can be any of several homo- or heteropolymers. Typical examples thereof include xylan, arabinan xyloglucan, arabinoxylan, arabinogalactan, glucuronoxylan, glucomannan and galactomannan. Monomeric components of hemicellulose include, but are not limited to: D-glucose, D-galactose, L-galactose, D-mannose, L-rhamnose, L-fucose, D-xylose, L-arabinose, and D-glucuronic acid. This class of polysaccharides is found in almost all cell walls along with cellulose. Hemicellulose is lower in weight than cellulose and can be extracted by pressurized hot water, aqueous alkali and/or acid, enzymatic treatment, etc. Polymeric chains of hemicellulose bind pectin and cellulose in a network of cross-linked fibers forming the cell walls of most plant cells.

The content of various sugars and uronic acids referred to herein, including the content of arabinose, rhamnose, galactose, glucose, xylose, fucose, fructose, sucrose, glucuronic acid and galacturonic acid, can be determined by chromatographic analysis. A suitable method to determine the content of various sugars and uronic acids referred to herein, including the content of arabinose, rhamnose, galactose, glucose, xylose, fucose, fructose, sucrose, glucuronic acid and galacturonic acid, is by anion-exchange chromatography. A preferred method to determine the content of various sugars and uronic acids referred to herein, including the content of arabinose, rhamnose, galactose, glucose, xylose, fucose, fructose, sucrose, glucuronic acid and galacturonic acid, is in accordance with the monosaccharides and uronic acids determination protocol described herein. Thus, in embodiments, the methods and products of the invention are provided, having the content of various sugars and sugar acids referred to herein, such as the content of arabinose, rhamnose, galactose, glucose, xylose, fucose, fructose, sucrose, glucuronic acid and galacturonic acid, as described herein when determined in accordance with the monosaccharides and uronic acids determination protocol described herein.

A preferred method to measure dry matter content is in accordance with ICUMSA GS2/1/3/9-15 (2007) which is known to the person skilled in the art.

Protein content as referred to herein is determined using the Kjehldahl method with a conversion factor of 6.25.

As used herein, the term 'minerals' refers to chloride, bromide, nitrate, malate, sulfate, oxalate, phosphate, potassium, sodium, calcium and magnesium. As will be appreciated by those skilled in the art, these are minerals naturally present in the crude uronic acid composition to be purified in the methods as defined herein. Stated differently, these are minerals naturally present in the plant material that is hydrolysed to provide the aqueous liquid to be purified in the methods as defined herein. A suitable method to determine the mineral content, defined as the combined amount (i.e. the sum) of chloride, bromide, nitrate, malate, sulfate, oxalate, phosphate, potassium, sodium, calcium and magnesium, is by determining the anion content by chromatography, such as high pressure ion chromatography coupled to a conductivity detector (HPIC-CD) and by determining the cation content by inductively coupled plasma - atomic emission spectroscopy (ICP-AES) analysis. A preferred method to determine the mineral content is in accordance with the HPIC-CD protocol described herein (for the anions) and in accordance with the ICP-AES protocol described herein (for the cations). Thus, in embodiments, the methods and products of the invention are provided, having the mineral content characteristics as described herein when determined in accordance with the HPIC-CD protocol described herein (for the anions) and in accordance with the ICP-AES protocol described herein (for the cations). These methods are also used for determination of the content of individual ions.

As used herein the term "oligomeric first uronic acid" refers to the first uronic acid residues which are present in the composition as part of a polysaccharide (in the case of galacturonic acid, typically this is a pectin polysaccharide), but can be released upon extensive enzymatic treatment. Galacturonic acid is the backbone of the oligomer (o(1-4)-linked) leaving the acid as side-groups. A certain degree of these groups can be esterified. The amount of oligomeric first uronic acid referred to in the present document is an estimate and determined by enzymatically digesting the sample and measuring first uronic acid content in both the digested sample and a control sample (preferably with the monosaccharides and uronic acids determination protocol described herein), wherein the additional first uronic acid found in the enzymatically digested sample compared to the control sample is the oligomeric first uronic acid content. Preferably, the oligomeric first uronic acid content is determined according to the oligomeric uronic acid determination protocol described herein.

The content of various organic acids referred to herein, including the content of citric acid, malic acid, lactic acid, formic acid, acetic acid, propionic acid, pyrrolidone carboxylic acid, and butyric acid, can be determined by chromatographic analysis. A suitable method to determine the content of various organic acids referred to herein, including the content of citric acid, malic acid, lactic acid, formic acid, acetic acid, propionic acid, pyrrolidone carboxylic acid, and butyric acid, is by high pressure ion chromatography coupled with a conductivity detector (HPIC-CD). A preferred method to determine the content of various organic acids referred to herein, including the content of citric acid, malic acid, lactic acid, formic acid, acetic acid, propionic acid, pyrrolidone carboxylic acid, and butyric acid, is in accordance with the organic acid determination protocol described herein. Thus, in embodiments, the methods and products of the invention are provided, having the content of various organic acids referred to herein, including the content of citric acid, malic acid, lactic acid, formic acid, acetic acid, propionic acid, pyrrolidone carboxylic acid, or butyric acid, as described herein when determined in accordance with the organic acid determination protocol described herein.

A first aspect of the invention concerns a method of enriching a uronic acid containing composition, said method comprising the consecutive steps of:
a) providing a crude uronic acid composition comprising a first uronic acid and one or more monosaccharides;
b) subjecting the crude uronic acid composition of step a) to a chromatographic separation using a stationary phase comprising an anion exchange resin;
c) collecting an enriched uronic acid composition.

The term "enriching" as used herein should be interpreted to mean that the amount of uronic acid, based on dry matter, is increased. In as such, a refinement or purification of the uronic acid composition is achieved.

### The crude uronic acid composition

The crude uronic acid composition provided in step a) is an aqueous composition which comprises a first uronic acid and one or more monosaccharides. The composition may be provided in the form of a solution, or may comprise solid particles, for example dispersed plant particles. As explained herein before, the first uronic acid is preferably selected from the group consisting of galacturonic acid, mannuronic acid, guluronic acid, glucuronic acid, iduronic acid, and combinations thereof, most preferably the uronic acid is galacturonic acid.

The crude uronic acid composition will typically be a biomass hydrolysate, such as a hydrolysate of pectin or of pectin-containing biomass such as a plant material hydrolysate, algae hydrolysate or bacteria hydrolysate. Typically, and depending on the source, it will contain neutral sugars, such as arabinose, glucose, galactose, xylose, mannose, fructose and rhamnose; organic acids such as citric acid, malic acid, lactic acid, formic acid, acetic acid, propionic acid, pyrrolidone carboxylic acid, or butyric acid; minerals (particularly, K, Ca, Mg, Na, SO₄, PO₄, oxalate, CI), oligosaccharides and (soluble) polysaccharides.

Thus, in some embodiments the crude uronic acid composition provided in step a) comprises one or more organic acids other than the first uronic acid, preferably other than uronic acids. Preferably the one or more organic acids other than uronic acid is selected from citric acid, malic acid, lactic acid, formic acid, acetic acid, propionic acid, pyrrolidone carboxylic acid, butyric acid, and combinations thereof.

Typically, the crude uronic acid composition provided in step a) comprises at least 1 wt.% of the first uronic acid based on dry matter of the composition, preferably at least 5 wt.%, more preferably at least 10 wt.%. In a preferred embodiment of the invention, the first uronic acid is present in the composition at a level of at least 10 wt.%, based on dry matter of the composition, at least 15 wt.%, at least 17.5 wt.% or at least 20 wt.%. The crude uronic acid composition typically comprises less than 85 wt.% of the first uronic acid, based on dry matter of the composition. Depending on the origin of the crude uronic acid the level of the first uronic acid may vary strongly. For example, a pectin hydrolysate typically comprises more than 50 wt.% of the first uronic acid (in this case galacturonic acid), based on dry matter of the composition, while a plant material hydrolysate, algae hydrolysate or bacteria hydrolysate typically comprises less than 50 wt.% of the first uronic acid, based on dry matter of the composition. Thus, in some embodiments the crude uronic acid composition provided in step a) comprises 5-50 wt.% of the first uronic acid based on dry matter of the composition, preferably 10-45 wt.%, more preferably 15-40 wt.%. In some embodiments the crude uronic acid composition provided in step a) comprises 50-85 wt.% of the first uronic acid based on dry matter of the composition, preferably 50-80 wt.%, more preferably 55-75 wt.%.

Typically, the crude uronic acid composition provided in step a) contains neutral sugars (monosaccharides) at a combined level of 5-70 wt.%, based on dry matter of the composition. In a preferred embodiment of the invention, the neutral sugars (monosaccharides) are present in the crude composition at a combined level, based on dry matter, of at least 5 wt.%, preferably at least 10 wt.%, more preferably at least 30 wt.% and/or at a combined level, based on dry matter, of below 70 wt.%. In some scenarios, for example in case cellulose is not hydrolyzed, the amount of sugars will be lower, for example below 40 wt.%, based on dry matter. In a preferred embodiment of the invention, said neutral sugars (monosaccharides) are selected from the group consisting of fucose, rhamnose, arabinose, galactose, glucose, mannose, xylose, fructose, and sucrose.

Typically, in particular in case a beet pulp hydrolysate is provided as the crude uronic acid composition, the crude uronic acid composition provided in step a) comprises 10-40 wt.% arabinose, based on dry matter of the composition, preferably 20-40 wt.%. In a preferred embodiment of the invention, arabinose is present in the crude uronic acid composition, based on dry matter, at a level of at least 20 wt.%, preferably at least 22.5 wt.%, more preferably at least 25 wt.% and/or at a level of below 40 wt.%, preferably below 37.5 wt.%, preferably below 35 wt.%.

Typically, in particular in case cellulose was also hydrolyzed, the crude uronic acid composition provided in step a) comprises 10-40 wt.% glucose, based on dry matter of the composition, preferably 20-40 wt.%. In a preferred embodiment of the invention, glucose is present in the crude uronic acid composition, based on dry matter, at a level of at least 20 wt.%, preferably at least 22.5 wt.%, more preferably at least 25 wt.% and/or at a level of below 40 wt.%, preferably below 37.5 wt.%, preferably below 35 wt.%.

Typically, the crude uronic acid composition provided in step a) comprises a total combined amount of organic acids other than uronic acids, preferably organic acids selected from citric acid, malic acid, lactic acid, formic acid, acetic acid, propionic acid, pyrrolidone carboxylic acid, and butyric acid, of at least 0.1 wt.%, based on dry matter of the composition, preferably at least 0.5 wt.%, more preferably at least 1 wt.%. In a preferred embodiment of the invention, the total combined amount of organic acids other than the first uronic acid, preferably other thanuronic acids, preferably organic acids selected from citric acid, malic acid, lactic acid, formic acid, acetic acid, propionic acid, pyrrolidone carboxylic acid, and butyric acid in the crude uronic acid composition is at least 0.2 wt.%, at least 0.3 wt.%, at least 0.4 wt.% or at least 0.5 wt.% and/or below 20 wt.%, below 15 wt.%, or below 10 wt.% e.g. within the range of 0.1-20 wt.%, 0.2-15 wt.%, or within the range of 0.5-10 wt.%.

In certain embodiments, the crude uronic acid composition provided in step a) contains lactic acid at a level within the range of 0.1-15 wt.%, based on dry matter of the composition. In a preferred embodiment of the invention, lactic acid is present in the composition at a level of at least 0.1 wt.%, at least 0.5 wt.%, or at least 0.9 wt.% and/or at a level of below 15 wt.%, below 12.5 wt.%, or below 10 wt.%, e.g. at a level within the range of 0.6-15 wt.%, within the range of 0.1-12.5 wt.%, within the range of 0.5-12.5 wt.% or within the range of 0.9-10 wt.%. The lactic acid content of ensilaged plant material, such as ensilaged sugar beet pulp or chicory root pulp is typically relatively high because of lactic acid fermentation.

In certain embodiments, the crude uronic acid composition provided in step a) contains acetic acid at a level within the range of 0.1-10 wt.%, based on dry matter of the composition. In a preferred embodiment of the invention, acetic acid is present in the composition at a level of at least 0.1 wt.%, at least 0.5 wt.%, or at least 0.9 wt.% and/or at a level of below 10 wt.%, below 5 wt.% or below 3 wt.%, e.g. at a level within the range of at least 0.1-10 wt.%, within the range of 0.5-5 wt.%, or within the range of 0.9-3 wt.%.

In certain embodiments, in particular in case a sugar beet pulp hydrolysate is provided as the crude uronic acid composition, the crude uronic acid composition provided in step a) contains at least 0.01 wt.% ferulic acid, based on dry matter, preferably at least 0.05 wt.%, more preferably at least 0.25 wt.%. The amount of ferulic acid in the crude uronic acid composition is preferably less than 3.5 wt.%, based on dry matter, preferably less than 1 wt.%.

In accordance with highly preferred embodiments of the invention, the crude uronic acid composition provided in step a) comprises polysaccharides. In the context of the present invention, the term polysaccharides should be interpreted to denote any polysaccharide having two or more monosaccharide units. Typically, the crude uronic acid composition will comprise at least 0.05 wt.% of polysaccharides, based on dry matter, preferably at least 0.1 wt.%, preferably at least 0.3 wt.%. As is shown in the appended examples, it was surprisingly found that separation of the first uronic acid from polysaccharides, in particular from oligomeric first uronic acid is enabled by the methods of the present invention. Thus, in highly preferred embodiments of the invention the crude galacturonic acid composition provided in step a) comprises oligomeric galacturonic acid, preferably oligomeric first galacturonic acid. Typically, the crude uronic acid composition will comprise at least 0.05 wt.% of oligomeric first uronic acid, based on dry matter, preferably at least 0.1 wt.%, preferably at least 0.3 wt.%. The amount of oligomeric first uronic acid in the composition provided step a) is preferably below 10 wt.%, based on dry matter, more preferably below 6 wt.%, most preferably below 3 wt.%. The total combined amount of polysaccharides in the crude uronic acid composition is preferably below 10 wt.%, based on dry matter, preferably below 7.5 wt.%, more preferably below 5 wt.%.

Typically, in accordance with the invention, the protein level in the crude uronic acid composition is below 10 wt.%, based on dry matter of the composition. In a preferred embodiment of the invention, the protein level is below 7.5 wt.%, below 5 wt.%, below 4 wt.%, below 3 wt.%, below 2 wt.% or below 1 wt.%. In accordance with the invention, the crude uronic acid composition may be substantially or entirely free from proteins.

Typically, in the crude uronic acid composition, minerals are present at a (combined) level within the range of 0.1-12 wt.%, based on the total dry matter of the composition, preferably within the range of 0.1-8 wt.%, more preferably within the range of 0.1-5 wt.%.

The dry matter content of the crude uronic acid composition is not particularly limiting and may for example be 5-80 wt.%, preferably 10-65 wt.%, preferably 15-60 wt.%. In preferred embodiments of the invention, the crude uronic acid composition has a dry matter content of at least 20 wt.%, at least 30 wt.%, or at least 40 wt.% and/or below 70 wt.%, below 65 wt.%, or below 60 wt.%, e.g. within the range of 30-60 wt.%, or within the range of 35-55 wt.%

As explained herein before, the process of the invention is particularly suitable for the enrichment of uronic acid compositions obtained from a natural source. More preferably, the crude uronic acid composition comprises or is a biomass hydrolysate.

### Hydrolysate origin - pectin hydrolysates

The biomass hydrolysate may be a pectin hydrolysate, which can be prepared from previously extracted and purified pectin. In these embodiments the first uronic acid is galacturonic acid. In some embodiments of the invention, the process is provided wherein the biomass hydrolysate is citrus pectin hydrolysate, sugar beet pectin hydrolysate or chicory pectin hydrolysate. In the embodiments of the invention wherein the biomass hydrolysate is a pectin hydrolysate it is preferred that the hydrolysate further comprises glucose, wherein the ratio (w/w) of galacturonic acid to glucose in the crude uronic acid composition is more than 10:1, preferably more than 15:1. Such low amounts of glucose distinguish a pectin hydrolysate from hydrolysates comprising whole plant cell wall hydrolysis products.

In one embodiment of the invention, the crude uronic acid composition is a hydrolysate of pectin extracted from sugar beet and comprises:
- 40-70 wt.% based on dry matter of the composition, of galacturonic acid
- 0.5-30 wt.%, based on dry matter of the composition, of arabinose;
- 0.5-5 wt.%, based on dry matter of the composition, of glucose;
- 0.5-5 wt.%, based on dry matter of the composition, of lactic acid; and
- 0.5-5 wt.%, based on dry matter of the composition, of acetic acid.

In one embodiment of the invention, the crude uronic acid composition is a hydrolysate of pectin extracted from chicory and comprises:
- 40-80 wt.% based on dry matter of the composition, of galacturonic acid
- 0.5-0 wt.%, based on dry matter of the composition, of arabinose;
- 0.5-5 wt.%, based on dry matter of the composition, of glucose;
- 0.5-5 wt.%, based on dry matter of the composition, of lactic acid; and
- 0.5-5 wt.%, based on dry matter of the composition, of acetic acid.

### Hydrolysate origin - whole cell wall material hydrolysates

An important additional advantage in the context of the present invention is that the present enrichment methods allow good results to be achieved even on a complex hydrolysate such as those containing hydrolysis products of all cell wall polysaccharides, which contain large amounts of other components, in particular various sugars. This insight eliminates the need to first prepare pectin isolates. Thus, in preferred embodiments of the invention, the process is provided wherein the biomass hydrolysate comprises cell wall constituents. Such hydrolysates, which may also be referred to as "whole cell wall comprising hydrolysates" differ significantly from hydrolysates of a single cell wall component, such as pectin hydrolysates, since they comprise a much more elaborate and complex spectrum of components at much higher concentrations than in e.g. a pectin hydrolysate, the first uronic acid often not even being the major component in such a hydrolysate. The biomass hydrolysate comprising cell wall constituents referred to herein does not exclusively comprise cell wall constituents but may comprise further components arising from the fact that biomass comprising cell walls as well as other cell components of the biomass is used as a feedstock.

The biomass hydrolysate comprising cell wall constituents may be plant material hydrolysate, algae hydrolysate or bacteria hydrolysate.

In some embodiments of the invention, the biomass hydrolysate comprising cell wall constituents is plant material hydrolysate and further comprises glucose, wherein the ratio (w/w) of the first uronic acid to glucose in the crude uronic acid composition is less than 5:1, preferably less than 3:1, more preferably less than 2:1. In contrast to pectin hydrolysates, which are nearly devoid of glucose, the plant material hydrolysate comprising cell wall constituents has a high glucose content, reflecting that it is a hydrolysate comprising whole cell wall hydrolysis products, such as cellulose and hemicellulose hydrolysis products.

In some embodiments of the invention the biomass hydrolysate comprising cell wall constituents is a plant material hydrolysate comprising one or more, preferably two or more, preferably three or more compounds selected from the group consisting of arabinose, galactose, rhamnose, mannose, xylose, fructose, and fucose. In some embodiments the biomass hydrolysate comprising cell wall constituents is a plant material hydrolysate comprising arabinose, galactose, rhamnose, mannose, xylose, fructose, and fucose. This complex sugar composition further reflects that it is a hydrolysate comprising whole cell wall constituents. Thus in some preferred embodiments of the invention the biomass hydrolysate comprising cell wall constituents is a plant material hydrolysate which comprises glucose, wherein the ratio (w/w) of the first uronic acid to glucose in the crude uronic acid composition is less than 5:1, preferably less than 3:1, more preferably less than 2:1, and which comprises one or more, preferably two or more, preferably three or more compounds selected from the group consisting of arabinose, galactose, rhamnose, mannose, xylose, fructose, and fucose.

Arabinose is a plant-specific sugar accounting for 5-25% of cell wall constituents in plant material. In preferred embodiments of the invention, the biomass hydrolysate comprising cell wall constituents is a plant material hydrolysate further comprising arabinose and wherein the ratio (w/w) of the first uronic acid to arabinose in the crude uronic acid composition is less than 5:1, preferably less than 3:1, more preferably less than 2:1 and most preferably less than 1:1. In some preferred embodiments of the invention the biomass hydrolysate comprising cell wall constituents is a plant material hydrolysate which comprises glucose, wherein the ratio (w/w) of the first uronic acid to glucose in the crude uronic acid composition is less than 5:1, preferably less than 3:1, more preferably less than 2:1, and which comprises arabinose and wherein the ratio (w/w) of the first uronic acid to arabinose in the crude uronic acid composition is less than 5:1, preferably less than 3:1, more preferably less than 2:1 and most preferably less than 1:1.

Suitable plant material hydrolysates comprising cell wall constituents for use in the process according to the present invention are typically hydrolysates of plant materials mainly considered by-products in various industries. Such plant materials can be derived from the plant pulp, peel, seeds, fibres, skin and pomace. In preferred embodiments of the invention, in particular when the first uronic acid is galacturonic acid, the plant material hydrolysate comprising cell wall constituents is a hydrolysate of plant material, selected from spent sugar beet pulp, citrus pulp, whole pumpkin, pumpkin pulp, pumpkin shell, leek leaves, endive roots, whole cabbage, chicory pulp, onion hulls, whole parsley, endive leaves, apple cake, apple pomace, whole apple, pea pod, cucumber, berries, rapeseed press cake, seabuckthorn pulp, hop, olive pomace, tomato skins, whole tomato, grape pomace, whole pear, potato pulp, turnip, carrot, grape, whole pineapple, pineapple skin, cranberries, sunflower, rapeseed, lime, lemon, grapefruit, pear, apricot, guave, mango, plum, peach, quince, papaya, banana, coffee berry and combinations thereof, preferably hydrolysate of plant material selected from spent sugar beet pulp, chicory pulp, citrus pulp and combinations thereof, most preferably spent sugar beet pulp.

In preferred embodiments of the invention wherein the hydrolysate is an algae hydrolysate comprising cell wall constituents, the algae hydrolysate is obtained from brown, green or red algae. For brown seaweed in particular alginate containing brown macroalgae, such as species of Laminaria, Macrocystis, Ascophyllum, Eclonia, Lessonia, Durvillea, and Sargassum. For green seaweed, in particular ulvan containing green macroalgae like the family Ulvaceae, in particular *Ulva lactuca.* For red maroalgae, in particular those of the Florideophyceae class. In other embodiments of the invention, the algae hydrolysate is obtained from the group consisting of S. *fusiforme, Schizymenia dubyi, Serraticardia maxima, C. pilulifera, B. cretacea, Clathromorphum nereostratum, Amphiroa fragilissima, Corallina mediterranea, Vischeria punctata, Cyanidioschyzon merolae, Atractophora hypnoides, Gelidiella calcicole, Lemanea, Palmaria palmata, Schmitzia hiscockiana, Chondrus crisp us, Mastocarpus stellatus, Acrochaetium efflorescens, Audouinella, Polysiphonia ceramiaeformis,* and/or *Vertebrata simulans.*

In preferred embodiments of the invention, the hydrolysate is a bacterial biomass hydrolysate such as a bacterial biomass hydrolysate comprising bacterial alginate hydrolysate. The bacterial biomass hydrolysate may consist essentially of bacterial alginate hydrolysate, or may be provided in the form of a hydrolysate comprising cell wall constituents. The latter hydrolysate is obtainable when treating a biomass hydrolysate obtained from biomass comprising whole bacteria, such as when a biomass hydrolysate comprising a mixture of alginate and alginate producing bacteria is treated. Examples of suitable alginate producing bacteria are selected from Zotobacter vinelandii, A. chroococcum and Pseudomonas species which have been genetically modified to produce alginate.

### Integrated process

In some highly preferred embodiments of the present invention, the process comprises the steps of:
(i) providing biomass;
(ii) subjecting the biomass of step (i) to aqueous digestion to obtain a crude hydrolysate;
(iii) optionally subjecting the crude hydrolysate of step (ii) to a solid-liquid separation step to obtain a clarified biomass hydrolysate; and
(iv) optionally concentrating or diluting the crude or clarified biomass hydrolysate
(v) providing the optionally concentrated or diluted, crude or clarified, biomass hydrolysate as the crude uronic acid composition of step a).

In this way, an integrated process is achieved which starts from a biomass feedstock, typically a residual material, and allows obtaining a valuable enriched uronic acid compositions in a facile and efficient process. The biomass is a material as described herein before in the context of the hydrolysate properties.

The biomass, in particular plant material, can be subjected to one or more pre-treatment steps before it is submitted to step (ii). The pre-treatment facilitates the subsequent production of hydrolysate. Non-limiting examples of suitable pre-treatments are:
- a comminution step to reduce the size before it is submitted to step (ii), for example shredding, slicing, milling, cutting, mashing, etc.;
- one or more washing steps using neutral, acidic or basic aqueous compositions, for example washing employing water will facilitate the dissolution and removal of various salts, and free organic acids, like lactic acid;

- treatments resulting in cell wall disintegration or increased permeation thermal (e.g. steam explosion treatment), chemical (e.g. acid and/or base treatment) or mechanical treatments), optionally followed by extraction of a non-structural cell constituent (e.g. sucrose extraction from sugar beet, mannitol extraction from seaweed, inulin extraction from chicory);
- pulsed electric field (PEF) treatment;
- drying, e.g. freeze-drying and/or steam drying;
- fermentation including ensilage treatment;
- dewatering, e.g. via pressing; and/or
- enzymatic pre-treatment.

In case the process is performed using spent sugar beet pulp as biomass feedstock, it will generally already have been submitted to thermal cell disintegration pre-treatment in the traditional sucrose extraction process. If the spent sugar beet pulp was ensilaged, it will also have fermented.

As described herein before, the biomass is preferably spent sugar beet pulp derived from conventional sugar (sucrose) production. Particularly preferred is the use of pressed sugar beet pulp from which a major amount of sucrose has been extracted and which has a dry solids content of 10-50 wt.%, preferably 20-30 wt.%, for example approximately 25 wt.%. The pulp may have been ensilaged or dried.

Step (ii) may comprise any treatment suitable to reduce the average molecular weight of one or more polysaccharides comprised in the biomass, in particular suitable to reduce the average molecular weight of pectin comprised in the biomass in case a pectin-containing biomass like the plant materials described herein earlier is used. The aqueous digestion preferably comprises acid digestion and/or enzymatic digestion, preferably enzymatic digestion.

The acid digestion preferably comprises contacting the biomass with an aqueous solution of an acid, preferably a mineral acid, more preferably an aqueous solution of nitric acid, hydrochloric acid, sulfuric acid or phosphoric acid, even more preferably an aqueous solution comprising one of the mineral acids mentioned at a concentration of 1-3 % (w/v) and, typically, heating the solution to a temperature of e.g. at least 30 °C, at least 50 °C, at least 70 °C, at least 90 °C, or at least 100 °C, preferably under agitation, for a period long enough to liberate/extract most of the uronic acid. It is within the routine capabilities of those skilled in the art to determine and implement appropriate conditions for optimal acid hydrolysis.

The enzymatic digestion preferably comprises enzymatic digestion by one or more enzymes belonging to EC class 3.2.1 glycosidases, 4.2.2. lyases or 3.1.1. esterases. In some particular embodiments, the biomass is plant material as described herein earlier and the aqueous digestion comprises digestion by cellulase, pectinase, and pectin methylesterase. Additional enzymes or other agents may be used in step (ii) to facilitate digestion of the polysaccharides into monosaccharides or to facilitate hydrolysis of esterified sidegroups. Step (ii) can take place in the form of any aqueous composition. Depending on the dry matter content the composition will take the form of e.g. a dispersion, a slurry or pulp. The aqueous digestion of plant material of step (ii) is performed to increase the monosaccharide and/or uronic acid content of the plant material composition, thereby obtaining a hydrolysate. It is within the routine capabilities of those skilled in the art to adjust the process parameters to accomplish the desired rate and extent of polysaccharide depolymerisation (e.g. primarily cellulose, pectin and hemicellulose in case a plant material is being hydrolyzed). Useful guidance on the production of plant material hydrolysates, such as sugar beet pulp hydrolysates, can be found in Bioresour Technol 2013, 128, 518- 525.

In preferred embodiments of the invention, in particular in case step (ii) comprises enzymatic digestion, step (ii) is performed at a temperature within the range of 20 to 60 °C, preferably within the range of 35 to 55 °C, more preferably within the range of 40 to 50 °C.

In preferred embodiments of the invention, in particular in case step (ii) comprises enzymatic digestion, step (ii) is performed at a pH within the range of 3 to 5.

In preferred embodiments of the invention, in particular in case step (ii) comprises enzymatic digestion, step (ii) is performed at a dry matter content within the range of 2-40 wt.%, preferably within the range of 5-30 wt.%, most preferably within the range of 8-25 wt.%.

Hence, as will be understood from the above, step (ii) is preferably performed a temperature within the range of 20 to 60 °C, preferably within the range of 35 to 55 °C, more preferably within the range of 40 to 50 °C, and at a pH within the range of 3 to 5, and at a dry matter content within the range of 2-50 wt.%, preferably within the range of 5-30 wt.%, most preferably within the range of 8-20 wt.%.

Preferably step (iii) is performed.

Prior to subjecting the crude hydrolysate of step (ii) to the optional but preferred solid-liquid separation of step (iii), a denaturation step, typically by heating to a temperature of at least 50 °C, preferably at least 60 °C, more preferably at least 70 °C may be performed. As will be understood by the skilled person, the denaturation time, pH and temperature are interdependent. It is within the routine capabilities of the skilled person to achieve a desired extent of denaturation for the enzyme concerned. Such a denaturation step deactivates and typically coagulates the enzymes employed in step (ii) such that they also can be removed from the crude hydrolysate in step (iii). In some embodiments the denaturation step is performed. In other embodiments the denaturation step is not performed.

The solid-liquid separation of step (iii) can comprise any solid-liquid separation technique known to the skilled person, such as filtration, pressing, decantation, centrifugation, cyclone separation, etc., or combinations thereof. The solid-liquid separation is performed to remove a major amount of the fibres, insoluble ash (minerals), and/or coagulated proteins comprised in the crude hydrolysate.

In some embodiments of the invention, step (iii) comprises the steps:
(iiia) subjecting the crude hydrolysate of step (ii) to a solid-liquid separation step as detailed herein above and collecting the liquid fraction; and
(iiib) subjecting the liquid fraction collected in step (iiia) to a further purification and/or concentration step to obtain the clarified biomass hydrolysate.

The further purification and/or concentration step (iiib) is preferably performed to increase the first uronic acid concentration of the hydrolysate. The increase in first uronic acid concentration may be achieved based on total mass (e.g. when solvent is removed) and/or based on dry matter content (e.g. when carbohydrates other than the uronic acid are removed). The further purification and/or concentration step (iiib) may comprise ion-exchange treatment (e.g. softening), electrodialysis treatment and/or membrane filtration, solvent (typically water) evaporation, and/or reverse osmosis. Membrane filtration may comprise microfiltration, ultrafiltration and/or nanofiltration. As used herein, the term 'nanofiltration' (sometimes referred to in the art as ultrafiltration) refers to filtration using membranes with a pore size of less than 10 nm. As used herein, the term 'microfiltration' refers to filtration using membranes with a pore size of 0.1-10 µm. Membrane filtration preferably comprises ultrafiltration employing a membrane having a molecular weight cut-off value within the range of 0.5-50 kDa, preferably 1-20 kDa, more preferably 2-10 kDa (such as about 5 kDa) and collecting the permeate. In some embodiments of the invention, step (iii) comprises adjusting the pH of the hydrolysate. The pH is preferably adjusted to be within the range of 1-3.5, more preferably in the range of 1-2.5. Thus, in some embodiments, step (iii) comprises the steps:
(iiia) subjecting the crude hydrolysate of step (ii) to a solid-liquid separation step as detailed herein above and collecting the liquid fraction;
(iiib) subjecting the liquid fraction collected in step (iiia) to a further purification and/or concentration step to obtain a clarified biomass hydrolysate; and
(iiic) adjusting the pH of the crude hydrolysate obtained in step (ii), the liquid fraction of step (iiia) or the hydrolysate of step (iiib).

The present inventors have found that it is generally preferred if the crude uronic acid composition provided in step a) has been subjected to a softening step, in particular in case the crude uronic acid composition provided in step a) originates from spent sugar beet pulp. If the process comprises steps (i)-(v) explained herein before, the softening is typically performed as part of step (iiib). Softening reduces the Ca and Mg content and was found to lead to improved uronic acid yields, in particular because precipitation of Ca or Mg uronate salts is reduced or avoided. Spent sugar beet pulp typically comprises high Ca levels due to pressing aids employed in the process of extracting sugar from sugar beet, eventually giving rise to sugar beet pulp.

### Chromatographic separation

In accordance with the invention, the present method comprises the step b) of subjecting the crude uronic acid composition of step a) to a chromatographic separation using a stationary phase comprising an anion exchange resin.

As will be understood by those skilled in the art, the present invention concerns chromatographic separation. This is a well-known technique which typically employs a packed bed of resin material which is utilized in elution mode and, as such, is clearly distinguishable from anion exchange separation methods (such as those taught in the prior art), which are done in displacement mode, employing an adsorption stage wherein the resin is loaded with material to be adsorbed followed by a desorption stage wherein said material is desorbed by employing different process conditions (e.g. eluent composition) as in the adsorption stage.

Anion-exchange resins that are suitable for use in the present invention include resins known as weakly basic anion exchange resins and strongly basic anion exchange resins.

The resin is preferably a polymeric resin. Typically the resins comprise a polymeric backbone which has ion-exchanging sites introduced after polymerisation. The polymeric backbone is preferably selected from polystyrene, polystyrene and styrene copolymers, polyacrylate, aromatic substituted vinyl copolymers, polymethacrylate, phenol-formaldehyde, polyalkylamine, and combinations thereof. In an embodiment of the invention, the polymer backbone is selected from polystyrene and styrene copolymers, polyacrylate, and polymethacrylate. In an embodiment of the invention the polymer backbone is selected from styrene divinylbenzene copolymers or polyacrylate. In an embodiment of the invention, the ion-exchanging sites comprise tertiary or quaternary ammonium groups.

As the skilled person will understand, a tertiary ammonium group can reversibly exist in the form of an uncharged tertiary amine or in the form of a charged tertiary ammonium group through (de)protonation. The ionic form of a tertiary ammonium group in the resin will thus depend on the pretreatment of the resin and the operation conditions. In the methods described herein, an anion-exchange resin is used. Consequently it is preferred that a majority of the tertiary ammonium groups is in the cationic form. This is easily achieved by pretreatment of the resin with acid (such as a mineral acid) and/or using an acidic eluent. In some embodiments of the invention, the method comprises conditioning of the resin with a mineral acid as described herein elsewhere, preferably with sulfuric acid, before it is used in step b).

In accordance with the invention, the weak base resin stationary phase may be a gel-type (i.e. non-porous) or a microporous type, the gel-type being particularly preferred.

Typically, in accordance with the invention, the resin is provided in bead form, more preferably in the form of spherical beads. It is preferred that the beads are characterized by a D50 within the range of 10 to 2000 µm, more preferably from 100 to 1000 µm, 200 to 750 µm, 300 to 600 µm, or 400 to 500 µm, as determined using light obscuration size determination. The anion exchange resin is preferably provided in bead form, more preferably in the form of spherical beads, having a median diameter within the range of 10 to 2000 µm, more preferably from 100 to 1000 µm, 200 to 750 µm, 300 to 600 µm, or 400 to 500 µm. Furthermore, in preferred embodiments, the anion exchange resin provided in (spherical) bead form has a uniformity coefficient, defined as the ratio of D60 to D10, of below 1.5, preferably below 1.4, more preferable below 1.3. The particle-size distribution characteristics referred to herein are expressed based on a number-based distribution.

In some embodiments, the anion exchange resin has a water retention capacity within the range of 50-70 %, more preferably, 52-68 %, 54-66 % or 56-64 % and/or a total exchange capacity of more than 1.2 eq/L, more preferably more than 1.3 eq/L, more than 1.4 eq/L, more than 1.5 eq/L or more than 1.6 eq/L.

In particularly preferred embodiments of the invention, the weak base resin used in step b) is Amberlite^{™} CR5550, Amberlite^{™} CR7300, or a resin with similar properties. Amberlite^{™} CR5550 is a resin having a crosslinked polyacrylate backbone with tertiary ammonium functional groups (if pretreated with an acid). It has a gel matrix and takes the form of spherical beads with a total exchange capacity of ≥1.6 eq/l, a water retention capacity of 56-64% and a particle diameter of 400-500 µm, with a uniformity coefficient of ≤1.3. Amberlite^{™} CR7300 is a resin having a styrene-divinylbenzene backbone with quaternary ammonium functional groups. It has a gel matrix and takes the form of spherical beads with a total exchange capacity of ≥1.3 eq/l, a water retention capacity of 49-55% and a particle diameter of 280-340 µm, with a uniformity coefficient of ≤1.15.

Suitable examples of other resins that may be used in the present invention include Amberlite^{™} CG-4B, Amberlite^{™} IRA-35, Diaion^{™} WA-30, Diaion^{™} WA-20, Diaion^{™} WA-10, Amberlite^{™} IRA-93, Amberlite^{™} IRA-68, Amberlyst^{™} A-21, Amberlyst^{™} A-23, Amberlyst^{™} A-24, Amberlite^{™} IRA-92, Amberlite^{™} IRA-95, Amberlite^{™} IRA-96, Dowex^{™} 66, Lewatit^{™} MP-62, Marathon^{™} WBA, Marathon^{™} WBA-2, Monosphere^{™} 66, Monosphere^{™} 66/77. References to tradenames herein should be construed as referring to the product marketed under that tradename on 01 December 2023.

In accordance with the present invention, step b) is typically carried out using an acidic mobile phase, preferably an acidic aqueous mobile phase. In preferred embodiments of the invention the mobile phase used in step b) has a pH at which the first uronic acid is substantially in the non-dissociated form, e.g. the pH is within the range of 1-3.5, preferably within the range of 1-2.5. In some embodiments the pH is at least 1, preferably at least 1.4 and/or below 2.5, preferably below 2.25, below 2, below 1.8, below 1.7, below 1.6, or below 1.5. In any case it is highly preferred if the pH is below the first pKa of the first uronic acidin the case of galacturonic acid thus below pH 3.5. In a particularly preferred embodiment the mobile phase used in step b) has a pH in the range of 1.2-1.6, preferably of around 1.4. In highly preferred embodiments of the invention the mobile phase comprises a mineral acid, preferably a mineral acid selected from the group consisting of sulfuric acid, hydrochloric acid, phosphoric acid, and nitric acid. As will be understood by the skilled person, a strong mineral acid (such as the ones listed here) dissociates essentially completely upon contact with water, such that a mobile phase comprising a mineral acid should be construed to refer to a mobile phase obtained upon dissolution of the mineral acid. In particularly preferred embodiments of the invention, the mobile phase used in step b) comprises sulfuric acid (H₂SO₄). Without wishing to be bound by any theory, the use of H₂SO₄ is particularly suitable to bring and maintain the functional groups of the resin in the protonated state, while H₂SO₄ while it does not result in substantial degradation of saccharide molecules. Typically, in accordance with the invention, the mobile phase used in step b) is an aqueous H₂SO₄ solution at a concentration of at least 0.1 wt.%, e.g. at least 0.125 wt.%, at least 0.15 wt.% at least 0.16 wt.%, at least 0.17 wt.%, at least 0.18 wt.%, at least 0.19 wt.% or at least 0.20 wt.% and/or at a concentration of less than 0.3 wt.%, e.g. less than 0.275 wt.%, less than 0.25 wt.%, less than 0.24 wt.%, less than 0.23 wt.%, less than 0.22 wt.%, less than 0.21 wt.% or less than 0.20 wt.%. For example, in preferred embodiments of the invention the mobile phase of step b) comprises 0.05-1 wt.% H₂SO₄, preferably 0.1-0.5 wt.%, more preferably 0.1-0.3 wt.%. The mobile phase is preferably substantially free of other components than water and the mineral acid.

It has been found that the temperature may affect the overall efficiency of the process, presumably due to its influence on the viscosity of the liquids within the system, although the inventors do not wish to be bound by any theory in this regard. In preferred embodiments of the invention, step b) is performed at a temperature within the range of 30-80 °C. In particularly preferred embodiments, step b) is performed at a temperature of at least 40 °C, at least 45 °C, at least 50 °C, at least 55 °C, at least 57.5 °C or at least 60 °C and/or at below 77.5 °C, below 75 °C, below 74 °C, below 73 °C, below 72 °C, below 71 °C or below 70 °C.

The present enrichment method is a preparative chromatographic method which can be carried out in any mode known to the skilled person, including pulsed or batch chromatography and continuous chromatography such as real or simulated moving bed chromatography. It is however particularly preferred to carry out the present method in the form of simulated moving bed chromatography (SMBC). Hence, in a particularly preferred embodiment of the invention, a method as defined herein is provided, wherein the chromatographic separation of step b) comprises simulated moving bed chromatography. SMBC is a continuous chromatographic separation technique using multiple columns connected to each other and is known to the skilled person. The continuous process improves separation performance and throughputs.Suitable SMBC systems can be obtained from various manufacturers and it is within the routine capabilities of those skilled in the art to operate them, based on the guidance provided in the present disclosure, so as to collect the enriched uronic acid compositions of the present invention.

Step c) of collecting an enriched uronic acid composition is the logical consequence of separating first uronic acid from other components in the chromatographic separation. In case of a noncontinuous chromatographic setup, the enriched uronic acid composition is collected as an eluent fraction at a timepoint when an enriched uronic acid composition is eluting from the column. Indeed, as is shown in the examples which employ a regular linear, pulsed chromatographic setup, the earlier fractions collected from the column are enriched for oligomeric first uronic acid and sugars, while the later fractions collected from the column are enriched for first uronic acid. This is reflected by a change in the ratio of first uronic acid to oligomeric first uronic acid when comparing the crude and enriched compositions. Thus, in preferred embodiments the method of the present invention is provided wherein A_{crude}:B_{crude} < A_{enriched}:B_{enriched} wherein
A_{crude} is the concentration of first uronic acid, based on dry matter, in the crude uronic acid composition;
B_{crude} is the concentration of oligomeric first uronic acid, based on dry matter, in the crude uronic acid composition;
A_{enriched} is the concentration of first uronic acid, based on dry matter, in the enriched uronic acid composition;
B_{enriched} is the concentration of oligomeric first uronic acid, based on dry matter, in the enriched uronic acid composition. The amount of oligomeric first uronic acid is determined as described herein elsewhere.

As is shown in the examples, even in a simple pulsed chromatography setup using a single column high separation efficiencies combined with high yields can be achieved if the optimal fractions of eluent are collected. Thus, in preferred embodiments of the invention the method is provided wherein A_{enriched}:B_{enriched} is more than 10 times A_{crude}:B_{crude}, preferably wherein A_{enriched}:B_{enriched} is more than 25 times A_{crude}:B_{crude}, more preferably A_{enriched}:B_{enriched} is more than 50 times A_{crude}:B_{crude}.

In preferred embodiments of the invention, the method is provided wherein more than 50 % of the total amount of first uronic acid contained in the crude uronic acid composition of step a) is collected in step c), preferably more than 70 %, more preferably more than 90 % or more than 95 %. As can be seen from the data in the appended examples, the method of the present invention allows very high separation efficiencies to be achieved between the first uronic acid and other components of the crude composition such as monosaccharides and oligomeric first uronic acid. Thus, aside from high yields, a high purity can be achieved. Hence, in preferred embodiments of the invention, the enriched uronic acid composition collected in step c) comprises at least 50 wt.% uronic acid, based on dry matter, preferably at least 70 wt.%, more preferably at least 90 wt.%.

In some embodiments of the invention:
- more than 50 % of the total amount of first uronic acid contained in the crude uronic acid composition of step a) is collected in step c), preferably more than 70 %, more preferably more than 90 % or more than 95 %; and
- the enriched uronic acid composition collected in step c) comprises at least 50 wt.% first uronic acid, based on dry matter, preferably at least 70 wt.%, more preferably at least 90 wt.%.

In preferred embodiments of the invention:
- more than 70 % of the total amount of first uronic acid contained in the crude uronic acid composition of step a) is collected in step c), preferably more than 90 %, more preferably more than 95 %; and
- the enriched uronic acid composition collected in step c) comprises at least 70 wt.% first uronic acid, based on dry matter, preferably at least 90 wt.%.

In highly preferred embodiments of the invention, the method is provided wherein:
- more than 50 % of the total amount of first uronic acid contained in the crude uronic acid composition of step a) is collected in step c), preferably more than 70 %, more preferably more than 90 % or more than 95 %; and
- the enriched uronic acid composition collected in step c) comprises at least 50 wt.% first uronic acid, based on dry matter, preferably at least 70 wt.%, more preferably at least 90 wt.%; and
- wherein A_{enriched}:B_{enriched} is more than 10 times A_{crude}:B_{crude}, preferably wherein A_{enriched}:B_{enriched} is more than 25 times A_{crude}:B_{crude}, more preferably A_{enriched}:B_{enriched} is more than 50 times A_{crude}:B_{crude}.

In even more preferred embodiments of the invention:
- more than 70 % of the total amount of first uronic acid contained in the crude uronic acid composition of step a) is collected in step c), preferably more than 90 %, more preferably more than 95 %; and
- the enriched uronic acid composition collected in step c) comprises at least 70 wt.% first uronic acid, based on dry matter, preferably at least 90 wt.%; and
- wherein A_{enriched}:B_{enriched} is more than 25 times A_{crude}:B_{crude}, preferably wherein A_{enriched}:B_{enriched} is more than 50 times A_{crude}:B_{crude}.

It is furthermore preferred that the method of the present invention is provided wherein A_{crude}:C_{crude} < A_{enriched}:C_{enriched} wherein
A_{crude} is the concentration of first uronic acid, based on dry matter, in the crude uronic acid composition;
C_{crude} is the total combined amount of fucose, rhamnose, arabinose, galactose, glucose, mannose, xylose, fructose, and sucrose, based on dry matter, in the crude uronic acid composition;
A_{enriched} is the concentration of first uronic acid, based on dry matter, in the enriched uronic acid composition;
C_{enriched} is the total combined amount of fucose, rhamnose, arabinose, galactose, glucose, mannose, xylose, fructose, and sucrose, based on dry matter, in the enriched uronic acid composition. In preferred embodiments of the invention, the method is provided wherein A_{enriched}:C_{enriched} is more than 10 times A_{crude}:C_{crude}, preferably wherein A_{enriched}:C_{enriched} is more than 25 times A_{crude}:C_{crude}, more preferably A_{enriched}:C_{enriched} is more than 50 times A_{crude}:C_{crude}.

In embodiments of the invention, the method is provided wherein:
- more than 50 % of the total amount of first uronic acid contained in the crude uronic acid composition of step a) is collected in step c), preferably more than 70 %, more preferably more than 90 % or more than 95 %; and
- the enriched uronic acid composition collected in step c) comprises at least 50 wt.% first uronic acid, based on dry matter, preferably at least 70 wt.%, more preferably at least 90 wt.%; and
- wherein A_{enriched}:B_{enriched} is more than 10 times A_{crude}:B_{crude}, preferably wherein A_{enriched}:B_{enriched} is more than 25 times A_{crude}:B_{crude}, more preferably A_{enriched}:B_{enriched} is more than 50 times A_{crude}:B_{crude}; and
- A_{enriched}:C_{enriched} is more than 10 times A_{crude}:C_{crude}, preferably wherein A_{enriched}:C_{enriched} is more than 25 times A_{crude}:C_{crude}, more preferably A_{enriched}:C_{enriched} is more than 50 times A_{crude}:C_{crude}.

In preferred embodiments of the invention:
- more than 70 % of the total amount of first uronic acid contained in the crude uronic acid composition of step a) is collected in step c), preferably more than 90 %, more preferably more than 95 %; and
- the enriched uronic acid composition collected in step c) comprises at least 70 wt.% first uronic acid, based on dry matter, preferably at least 90 wt.%; and
- wherein A_{enriched}:B_{enriched} is more than 25 times A_{crude}:B_{crude}, preferably wherein A_{enriched}:B_{enriched} is more than 50 times A_{crude}:B_{crude}, and
- A_{enriched}:C_{enriched} is more than 25 times A_{crude}:C_{crude}, preferably wherein A_{enriched}:C_{enriched} is more than 50 times A_{crude}:C_{crude}.

In some embodiments the method of the present invention is provided wherein A_{crude}:D_{crude} < A_{enriched}:D_{enriched} wherein
A_{crude} is the concentration of first uronic acid, based on dry matter, in the crude uronic acid composition;
D_{crude} is the total combined amount of glucuronic acid, citric acid, malic acid, lactic acid, formic acid, acetic acid, propionic acid, and butyric acid, based on dry matter, in the crude uronic acid composition;
A_{enriched} is the concentration of first uronic acid, based on dry matter, in the enriched uronic acid composition;
D_{ennched} is the total combined amount of glucuronic acid, citric acid, malic acid, lactic acid, formic acid, acetic acid, propionic acid, and butyric acid, based on dry matter, in the enriched uronic acid composition. In preferred embodiments of the invention, the method is provided wherein A_{enriched}:D_{enriched} is more than 10 times A_{crude}:D_{crude}, preferably wherein A_{enriched}:D_{enriched} is more than 10 times A_{crude}:D_{crude}, more preferably A_{enriched}:D_{enriched} is more than 50 times A_{crude}:D_{crude}.

In accordance with the invention, step c) comprises recovering an aqueous composition. This aqueous composition may be further concentrated. The concentrating may be performed by any process or combination of processes known in the art, such as membrane methods (e.g. reverse osmosis or nanofiltration employing a very low MWCO membrane), evaporation, precipitation, crystallization, spray-drying, freeze-drying or drum drying. In highly preferred embodiments, step (d) comprises or consists of evaporation, crystallization and/or spray-drying, preferably evaporation followed by crystallization or spray-drying.

In highly preferred embodiments of the invention, a majority of the first uronic acid comprised in the composition recovered in step c) is present in its free acid form, preferably more than 90 mol%, such as more than 95 mol% or more than 99 mol% of the uronic acid comprised in the composition recovered in step c) is present in its free acid form. Hence, the composition of step c) preferably has a pH of 1-3.5, preferably 1.5-2.5.

### Enriched uronic acid compositions

A second aspect of the invention concerns enriched or purified uronic acid compositions that are obtainable by the any one of the methods provided herein.

A further aspect of the invention concerns an enriched uronic acid composition, comprising
- at least 50 wt.% of a first uronic acid, based on dry matter, preferably at least 70 wt.%, more preferably at least 90 wt.%;
- at least 0.1 wt.% of monosaccharides selected from arabinose and glucose, based on dry matter, preferably at least 0.25 wt.%;
- less than 0.1 wt.% of oligomeric first uronic acid, based on dry matter, preferably less than 0.01 wt.%, more preferably less than 0.001 wt.%.

The first uronic acid is preferably selected from the group consisting of galacturonic acid, mannuronic acid, guluronic acid, glucuronic acid, iduronic acid, and combinations thereof, preferably the uronic acid is galacturonic acid.

In a preferred embodiment of the invention, the first uronic acid is present in the composition at a level, based on dry matter, of at least 60 wt.%, at least 65 wt.%, at least 70 wt.%, at least 75 wt.% and/or at a level of below 95 wt.%, below 92.5 wt.%, or below 90 wt.

Typically, the enriched uronic acid composition contains neutral sugars (monosaccharides) at a total combined level of 0.1-15 wt.%, based on dry matter of the composition. In a preferred embodiment of the invention, the neutral sugars (monosaccharides) are present in the crude composition at a total combined level, based on dry matter, of at least 0.5 wt.%, preferably at least 1 wt.%, more preferably at least 3 wt.% and/or at a level of below 12 wt.%, preferably below 10 wt.%, most preferably below 5 wt.%. In a preferred embodiment of the invention, said neutral sugars (monosaccharides) are selected from the group consisting of fucose, rhamnose, arabinose, galactose, glucose, mannose, xylose, fructose, and sucrose.

Typically, in particular in case a beet pulp hydrolysate is provided as the crude uronic acid composition, the enriched uronic acid composition contains arabinose at a level, based on dry matter, of at least 0.1 wt.%, preferably at least 0.25 wt.% and/or at a level of below 10 wt.%, preferably below 5 wt.%.

Typically, the enriched uronic acid composition contains glucose at a level based on dry matter, of at least 0.1 wt.%, preferably at least 0.25 wt.% and/or at a level of below 10 wt.%, preferably below 5 wt.%.

The enriched uronic acid composition may contain organic acids other than uronic acid, preferably organic acids selected from citric acid, malic acid, lactic acid, formic acid, acetic acid, propionic acid, pyrrolidone carboxylic acid, and butyric acid, for example at a combined level within the range of 0.1-20 wt.%, based on dry matter, preferably within the range of 0.5-5 wt.%.

Typically, in the enriched uronic acid composition, minerals are present at a (combined) level within the range of 0.1-3 wt.%, based on dry matter.

In a particularly preferred embodiment of the invention, the enriched composition comprises less than 100 mg/kg calcium, preferably less than 50 mg/kg, more preferably less than 10 mg/kg.

Typically, in accordance with the invention, the enriched uronic acid composition is obtained from the process in the form of an aqueous liquid. The amount of water can vary, as will be understood by those of average skill in the art and the composition may be further concentrated as described herein earlier.

In preferred embodiments of the invention, enriched uronic acid compositions are provided having a dry matter content within the range of 10-90 wt.%. For example, the dry matter content can be at least 20 wt.%, at least 30 wt.%, at least 35% wt.% or at least 40 wt.% and/or below 80 wt.%, below 70 wt.% or below 60 wt.%. In preferred embodiments of the invention, the enriched uronic acid containing compositions are provided in the form of aqueous solutions.

In some embodiments of the invention the enriched uronic acid compositions are provided in the form of a solid, such as a solid powder, preferably a spray-dried, crystallized or freeze-dried powder.

A further embodiment of the invention concerns a container comprising the uronic acid composition as provided above, in the form of a liquid (such as a solution or dispersion), at a quantity exceeding 1 liter, preferably exceeding 10 liter, exceeding 25 liter, exceeding 50 liter or exceeding 100 liter.

The properties described herein for the uronic acid compositions are equally applicable to the enriched uronic acid compositions obtainable by the present process.

### Sugar raffinate composition

In case the process of the invention is performed as a binary separation, as is typically the case when e.g. simulated moving bed chromatography is used, next to the enriched uronic acid composition described above, a composition enriched in sugar is also obtained (referred to hereinafter as "sugar raffinate composition"). In some embodiments of the invention, step c) further comprises collecting a sugar raffinate composition. The sugar raffinate composition has a higher relative amount of monosaccharides and oligomeric first uronic acid than the crude uronic acid composition and have a lower relative amount of first uronic acid than the crude uronic acid composition.

The present inventors have found that such enriched sugar compositions have certain advantages in particular when used for food applications. For example they have very low or no uronic acids, which are generally considered an undesirable food ingredient while they are enriched for oligomeric uronic acid content (i.e. pectin type oligomers) which are desirable prebiotics.

Thus, in another aspect of the invention there is provided the sugar raffinate composition obtainable from the process of the invention.

In a further aspect of the invention there is provided a sugar raffinate composition comprising
- less than 4 wt.% of a uronic acid selected from the group consisting of galacturonic acid, mannuronic acid, guluronic acid, glucuronic acid, iduronic acid, and combinations thereof, preferably galacturonic acid, based on dry matter, preferably less than 2 wt.%, more preferably less than 1 wt.%;
- at least 50 wt.% of monosaccharides selected from arabinose and glucose, based on dry matter, preferably at least 70 wt.%, more preferably at least 80 wt.%;
- more than 0.5 wt.% of the total combined amount of oligomeric uronic acids from uronic acids selected from the group consisting of galacturonic acid, mannuronic acid, guluronic acid, glucuronic acid, iduronic acid, and combinations thereof, preferably of galacturonic acid based on dry matter, preferably more than 0.75 wt.%, more preferably more than 1 wt.%.

The amount of a uronic acid selected from the group consisting of galacturonic acid, mannuronic acid, guluronic acid, glucuronic acid, iduronic acid, and combinations thereof, preferably galacturonic acid, comprised in the sugar raffinate composition may be very low in case an efficient chromatographic setup was used, such as less than 0.5 wt.%, based on dry matter, less than 0.1 wt.% or less than 0.01 wt.%. In practice, since the obtained purity needs to be balanced with obtained yield for a process to be economically viable, the amount of a uronic acid selected from the group consisting of galacturonic acid, mannuronic acid, guluronic acid, glucuronic acid, iduronic acid, and combinations thereof, preferably galacturonic acid,comprised in the sugar raffinate composition is preferably more than 0.5 wt.%.

The total combined amount of uronic acids selected from the group consisting of galacturonic acid, mannuronic acid, guluronic acid, glucuronic acid, iduronic acid, and combinations thereof in the sugar raffinate composition is preferably less than 4 wt.%, based on dry matter, preferably less than 2 wt.%, more preferably less than 1 wt.%. The total combined amount of uronic acids selected from the group consisting of galacturonic acid, mannuronic acid, guluronic acid, glucuronic acid, iduronic acid, and combinations thereof comprised in the sugar raffinate composition may be very low in case an efficient chromatographic setup was used, such as less than 0.5 wt.%, based on dry matter, less than 0.1 wt.% or less than 0.01 wt.%. In practice, since the obtained purity needs to be balanced with obtained yield for a process to be economically viable, the amount of total combined amount of uronic acids selected from the group consisting of galacturonic acid, mannuronic acid, guluronic acid, glucuronic acid, iduronic acid, and combinations thereof comprised in the sugar raffinate composition is preferably more than 0.5 wt.%.

Typically, the sugar raffinate composition contains neutral sugars (monosaccharides) at a total combined level of 55-98 wt.%, based on dry matter of the composition, preferably 60-96 wt.%, more preferably 80-95 wt.%, such as 85-95 wt.%. In a preferred embodiment of the invention, said neutral sugars (monosaccharides) are selected from the group consisting of fucose, rhamnose, arabinose, galactose, glucose, mannose, xylose, fructose, and sucrose.

The sugar raffinate composition may contain organic acids other than uronic acids, preferably organic acids selected from citric acid, malic acid, lactic acid, formic acid, acetic acid, propionic acid, pyrrolidone carboxylic acid, and butyric acid, for example at a combined level within the range of 0.1-20 wt.%, based on dry matter, preferably within the range of 0.5-8 wt.%.

Typically, in the sugar raffinate composition, minerals are present at a (combined) level within the range of 0.5-15 wt.%, based on dry matter, preferably 4-12 wt.%.

Typically, in accordance with the invention, the sugar raffinate composition is obtained from the process in the form of an aqueous liquid. The amount of water can vary, as will be understood by those of average skill in the art and the composition may be further concentrated as described herein earlier.

In preferred embodiments of the invention, sugar raffinate compositions are provided having a dry matter content within the range of 10-90 wt.%. For example, the dry matter content can be at least 20 wt.%, at least 30 wt.%, at least 35% wt.% or at least 40 wt.% and/or below 80 wt.%, below 70 wt.% or below 60 wt.%. In preferred embodiments of the invention, the sugar raffinate containing compositions are provided in the form of aqueous solutions.

In some embodiments of the invention the sugar raffinate compositions are provided in the form of a solid, such as a solid powder, preferably a spray-dried, crystallized or freeze-dried powder.

A further embodiment of the invention concerns a container comprising the sugar raffinate composition as provided above, in the form of a liquid (such as a solution or dispersion), at a quantity exceeding 1 liter, preferably exceeding 10 liter, exceeding 25 liter, exceeding 50 liter or exceeding 100 liter.

The properties described herein for the sugar raffinate compositions are equally applicable to the enriched sugar raffinate compositions obtainable by the present process.

The invention has been described by reference to certain embodiments discussed above. It will be recognized that these embodiments are susceptible to various modifications and alternative forms well known to those of skill in the art.

Many modifications in addition to those described above may be made to the structures and techniques described herein without departing from the spirit and scope of the invention. Accordingly, although specific embodiments have been described, these are examples only and are not limiting upon the scope of the invention.

All patent and literature references cited in the present specification are hereby incorporated by reference in their entirety.

The following examples are offered for illustrative purposes only, and are not intended to limit the scope of the present invention in any way.

### Examples

The concentrations of the different components provided in the experimental section and anywhere else herein, have been determined in accordance with the below analysis protocols.

*Monosaccharides and uronic acids determination protocol:* An ICS-3000 Ion Chromatography HPLC system equipped with a Dionex CarboPac PA-1 column (2 × 250 mm) in combination with a Dionex CarboPac PA-1 guard column (2 × 25 mm) and a pulsed electrochemical detector in pulsed amperometric 10 detection mode was used (ThermoFisher Scientific, Breda, The Netherlands). A flow rate of 0.25 mL min⁻¹ was used, the column was equilibrated with H₂O, elution was performed as follows: 0-40 min, H₂O; 40-55 min, 0-40% 1 M sodium acetate in 100 mM NaOH; 55-60 min, 1 M sodium acetate in 100 mM NaOH; 60-65 min, 150 mM NaOH; 75-90 min, H₂O, and detection of the monosaccharides or uronic acids was performed after post-column addition of 0.5 M sodium hydroxide (0.15 mL min⁻¹), with elution performed at 20 °C, wherein the concentration of monosaccharides and uronic acids in the solution was determined from calibration curves, using deoxygalactose as an internal standard.

*Oligomeric uronic acid determination protocol:* Oligomeric uronic acid is determined based on the additional uronic acid released upon extensive enzymatic hydrolysis such that all uronic acids present in polymerized form are released as uronic acids. 1 gram of sample was added to 100 ml water, 10 ml acetate buffer of pH 4.5 and 100 µl enzyme preparation was added. The resulting mixture was shaken at 45 °C for 48 hours, cooled and filtered using a 0.45 µm membrane filter. In parallel, a control sample was prepared which was treated in the same way, but without addition of enzymes. The enzyme to be used can be determined by the skilled person depending on the uronic acid to be analyzed, for example in case of oligomeric galacturonic acid determination Pectinex Ultra SP (Novo Nordisk) was used. Next, the monosaccharides and uronic acids determination protocol is performed on both the enzymatically digested sample and the control sample, and the difference in uronic acid content is calculated. The additional uronic acid found in the enzymatically digested sample compared to the control sample for each uronic acid and is the oligomeric uronic acid content of that uronic acid.

*Organic acid determination protocol:* The concentration of organic acids including the content of citric acid, malic acid, lactic acid, formic acid, acetic acid, propionic acid, pyrrolidone carboxylic acid, and butyric acid was determined using high pressure ion chromatography coupled with a conductivity detector (HPIC-CD). Measurements were performed using the following setup.

| | |
|---|---|
| Autosampler | Thermo AS-AP |
| Injection volume | 25 µL (full loop) |
| Injector temperature | 15 °C |
| Pump flow | 0.600 ml/min |
| Column | Biorad Aminex HPX-87H, 300 x 7.8 mm |
| Max. pressure | 1500 psi |
| Column temperature | 45 °C |
| Supressor | 10 mM ammoniumhydroxide |
| Supressor current | 2.00 mL/min |
| Detector | 35 °C |
| Detector temperature | 27 min |

| Step | Time (min) | Concentration (mM KOH |
|---|---|---|
| 1 | 0 | 10 |
| 2 | 8 | 10 |
| 3 | 21 | 26.25 |
| 4 | 33 | 50 |
| 5 | 33 | 75 |
| 6 | 35 | 75 |
| 7 | 35 | 10 |
| 8 | 37.5 | 10 |

*HPIC-CD protocol:* The concentration of chloride, bromide, nitrate, malate, sulfate, oxalate and phosphate were determined using high pressure ion chromatography coupled with a conductivity detector (HPIC-CD). A calibration curve was constructed by analysing a series of dilutions of a stock solution containing chloride, bromide, nitrate, phosphate, malate, sulfate and oxalate (100 mg/kg each in demineralized water). Measurements were performed using the following setup (employing a KOH gradient in the eluent).

| | |
|---|---|
| Autosampler | Thermo AS-AP |
| Injection volume | 7 µL |
| Injector temperature | 15 °C |
| Pump flow | 0.38 ml/min |
| Eluent generator | EGC 500 KOH |
| Column | Dionex IonPac AS11-HC-4µm |
| Max. pressure | 5000 psi |
| Column temperature | 35 °C |
| Supressor | AERS-500 2mm |
| Supressor current | 52 mA |
| Detector | Thermo Fisher Scientific Integrion Analytical CD |
| Detector temperature | 30 °C |
| Analysis time | 37.5 min |

| Step | Time (min) | Concentration (mM KOH) |
|---|---|---|
| 1 | 0 | 10 |
| 2 | 8 | 10 |
| 3 | 21 | 26.25 |
| 4 | 33 | 50 |
| 5 | 33 | 75 |
| 6 | 35 | 75 |
| 7 | 35 | 10 |
| 8 | 37.5 | 10 |

*ICP-AES protocol:* sodium, potassium, calcium and magnesium concentrations were determined using inductively coupled plasma - atomic emission spectroscopy (IPC-AES) analysis. Analyte samples were acidified using HNOs and measured in a type Thermo Fisher Scientific iCap 6000 series ICP emission spectrometer using 1150W RF power. The following solutions (acidified using HNO₃) were used for calibration purposes.

| Stock solution | Ca (mg/kg) | K (mg/kg) | Mg (mg/kg) | Na (mg/kg) |
|---|---|---|---|---|
| blank | 0 | 0 | 0 | 0 |
| 1 | 1.25 | 25 | 1.25 | 25 |
| 2 | 2.50 | 50 | 2.50 | 50 |
| 3 | 3.75 | 100 | 3.75 | 100 |
| 4 | 5 | 150 | 5 | 150 |

### Example 1: galacturonic acid from hydrolysate of sugar beet pulp

A sugar beet pulp hydrolysate was prepared by enzymatic hydrolysis of ensiled sugar beet pulp using pectinase and cellulase. The hydrolysate was clarified by the following steps: decanter centrifuge, disk stack centrifuge, ultrafiltration (5 kDa membrane). The permeate from the UF was concentrated by evaporation. The resulting concentrate was used as the crude uronic acid composition which is fed to the chromatographic separation step.

The composition of the crude uronic acid composition was as follows. The amounts are based on the total weight of the solution. The dry matter content was 45.7 wt.%.

| | |
|---|---|
| Galacturonic acid | Monomeric 11.38 wt.% |
| | Oligomeric 0.55 wt.% |
| Monosaccharides and other uronic acids | Arabinose 12.55 wt.% |
| | Glucose 12.53 wt.% |
| | Galactose 1.65 wt.% |
| | Fructose 1.03 wt.% |
| | Rhamnose 0.49 wt.% |
| | Xylose 0.31 wt.% |
| | Mannose 0.16 wt.% |
| | Sucrose 0.15 wt.% |
| | Fucose 0.06 wt.% |
| | Glucuronic acid 0.05 wt.% |
| Organic acids | Lactic acid 13000 mg/kg |
| | Acetic acid 2700 mg/kg |
| | Pyrrolidone carboxylic acid (PCA) 140 mg/kg |
| | Butyric acid 130 mg/kg |
| | Formic acid 58 mg/kg |
| | Citric acid 57 mg/kg |
| | Malic acid 51 mg/kg |
| | Propionic acid <40 mg/kg |
| Cations | Calcium 4700 mg/kg |
| | Potassium 2400 mg/kg |
| | Magnesium 1300 mg/kg |
| | Sodium 240 mg/kg |
| Anions | Sulphate 4500 mg/kg |
| | Phosphate 1600 mg/kg |
| | Oxalate 210 mg/kg |
| | Chloride 140 mg/kg |
| | Nitrate <20 mg/kg |
| | Bromide <20 mg/kg |

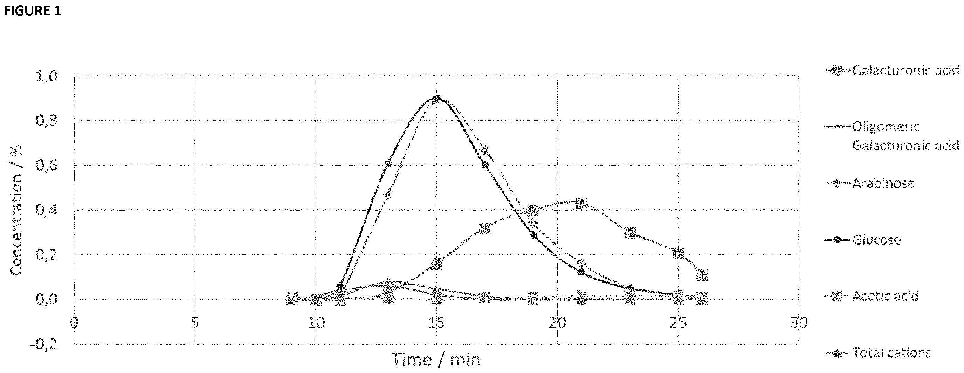

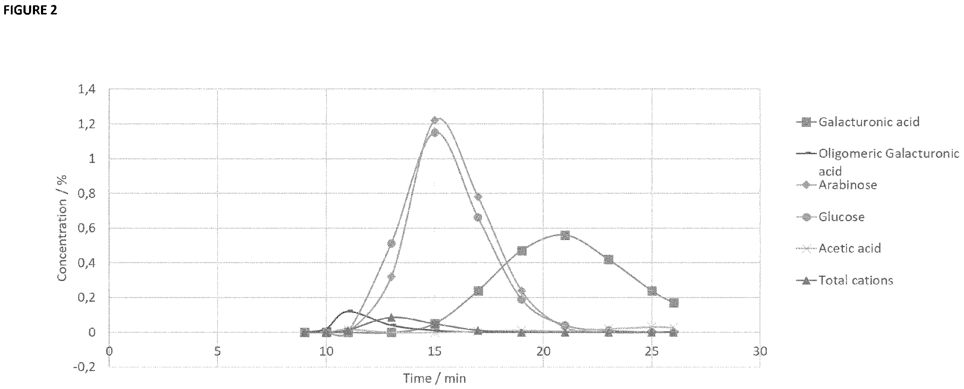

Next, the crude uronic acid composition was enriched or purified using the following chromatographic setup:
Column used: 600 × 10 mm
Jacketed
Flow: 2 mL/min
T: 60 °C
Injection time: 0.5 minute
0.2% H₂SO₄ as eluent
Dextran was used to determine system volume (t0) (9.5 min)
Resins were first conditioned in 4% H₂SO₄ before loading into the column
The test was run twice with different resins. The resins Amberlite^{™} CR5550 and Amberlite^{™} CR7300 were used. The results are shown in the tables below and figures 1 and 2. A value of "0" indicates below the detection limit. Empty cells indicate no measurement was made.

| **Amberlite^{™} CR5550** | | Run time / min | | | | | | | | | | |
|---|---|---|---|---|---|---|---|---|---|---|---|---|
| | | **9** | **10** | **11** | **13** | **15** | **17** | **19** | **21** | **23** | **25** | **26** |
| Potassium | mg/kg | 0 | 0 | 96 | 340 | 57 | 0 | | | 0 | 0 | |
| Sodium | mg/kg | 0 | 0 | 0 | 0 | 0 | 0 | | | 0 | 0 | |
| Calcium | mg/kg | 0 | 3..0 | 40 | 310 | 330 | 120 | | | 5.7 | 2.2 | |
| Magnesium | mg/kg | 0 | 2.8 | 28 | 120 | 74 | 22 | | | 1.1 | 0 | |
| Fucose | wt.% | 0 | 0 | 0 | 0 | 0 | 0 | 0 | 0 | 0 | 0 | 0 |
| Rhamnose | wt.% | 0 | 0 | 0 | 0.02 | 0.03 | 0.02 | 0.01 | 0.01 | 0 | 0 | 0 |
| Arabinose | wt.% | 0 | 0 | 0.02 | 0.47 | 0.89 | 0.67 | 0.34 | 0.16 | 0.05 | 0.02 | 0.01 |
| Galactose | wt.% | 0 | 0 | 0.01 | 0.08 | 0.12 | 0.07 | 0.03 | 0.01 | 0 | 0 | 0 |
| Glucose | wt.% | 0 | 0 | 0.04 | 0.57 | 0.89 | 0.60 | 0.30 | 0.14 | 0.05 | 0.02 | 0.01 |
| Mannose | wt.% | 0 | 0 | 0 | 0.01 | 0.01 | 0.01 | 0 | 0 | 0 | 0 | 0 |
| Xylose | wt.% | 0 | 0 | 0 | 0.01 | 0.02 | 0.02 | 0.01 | 0 | 0 | 0 | 0 |
| Fructose | wt.% | 0 | 0 | 0.01 | 0.06 | 0.08 | 0.05 | 0.02 | 0.01 | 0 | 0 | |
| Sucrose | wt.% | 0 | 0 | 0 | 0 | 0 | 0 | 0 | 0 | 0 | 0 | 0 |
| Galacturonic acid | wt.% | 0.01 | 0 | 0 | 0.03 | 0.16 | 0.32 | 0.40 | 0.43 | 0.30 | 0.21 | 0.11 |
| Glucuronic acid | wt.% | 0 | 0 | 0 | 0 | 0 | 0 | 0 | 0 | 0 | 0 | 0 |
| Oligomeric galacturonic acid | wt.% | 000 | 0.01 | 0.04 | 0.06 | 0.02 | 0.00 | 0.00 | 0.00 | 0.00 | 0.00 | 0.00 |
| Citric acid | mg/kg | 0 | 0 | 0 | 0 | 0 | 0 | 0 | 0 | 0 | 0 | 0 |
| Malic acid | mg/kg | 0 | 0 | 0 | 0 | 0 | 0 | 0 | 0 | 0 | 0 | 0 |
| Lactic acid | mg/kg | 0 | 0 | 0 | 0 | 0 | 89 | 210 | 380 | 400 | 400 | 240 |
| Formic acid | mg/kg | | 0 | 0 | 0 | 0 | 0 | 0 | 0 | 0 | 0 | 0 |
| Acetic acid | mg/kg | | | 88 | 52 | 0 | 73 | 100 | 160 | 160 | 170 | 89 |
| Pyrrolidone carboxylic acid (PCA) | mg/kg | 0 | 0 | 0 | 0 | 0 | 0 | 0 | 0 | 0 | 0 | 0 |
| Propionic acid | mg/kg | | | 0 | 0 | 0 | 0 | 0 | 0 | 0 | 0 | 0 |
| Butyric acid | mg/kg | 0 | 0 | 0 | 0 | 0 | 0 | 0 | 0 | 0 | 0 | 0 |

| **Amberlite^{™} CR7300** | | Run time / min | | | | | | | | | | |
|---|---|---|---|---|---|---|---|---|---|---|---|---|
| | | **9** | **10** | **11** | **13** | **15** | **17** | **19** | **21** | **23** | **25** | **26** |
| Potassium | mg/kg | 0 | 0 | 88 | 340 | 0 | 0 | | | 0 | 0 | |
| Sodium | mg/kg | 0 | 0 | 0 | 0 | 0 | 0 | | | 0 | 0 | |
| Calcium | mg/kg | 0 | 0.55 | 12 | 370 | 410 | 90 | | | 1.60 | 1.20 | |
| Magnesium | mg/kg | 0 | 0 | 20 | 140 | 73 | 14 | | | 0 | 0 | |
| Fucose | wt.% | 0 | 0 | 0 | 0 | 0.01 | 0 | 0 | 0 | 0 | 0 | 0 |
| Rhamnose | wt.% | 0 | 0 | 0 | 0.02 | 0.05 | 0.03 | 0.01 | 0 | 0 | 0 | 0 |
| Arabinose | wt.% | 0 | 0 | 0 | 0.32 | 1.22 | 0.78 | 0.24 | 0.03 | 0 | 0 | 0 |
| Galactose | wt.% | 0 | 0 | 0 | 0.09 | 0.15 | 0.07 | 0.02 | 0 | 0 | 0 | 0 |
| Glucose | wt.% | 0 | 0 | 0 | 0.51 | 1.15 | 0.66 | 0.19 | 0.04 | 0.01 | 0 | 0 |
| Mannose | wt.% | 0 | 0 | 0 | 0.01 | 0.02 | 0.01 | 0 | 0 | 0 | 0 | 0 |
| Xylose | wt.% | 0 | 0 | 0 | 0 | 0.02 | 0.02 | 0.01 | 0 | 0 | 0 | 0 |
| Fructose | wt.% | 0 | 0 | 0 | 0.06 | 0.11 | 0.05 | 0 | 0 | 0 | 0 | 0 |
| Sucrose | wt.% | 0 | 0 | 0 | 0 | 0 | 0 | 0 | 0 | 0 | 0 | 0 |
| Galacturonic acid | wt.% | 0 | 0 | 0 | 0 | 0.05 | 0.24 | 0.47 | 0.56 | 0.42 | 0.24 | 0.17 |
| Glucuronic acid | wt.% | 0 | 0 | 0 | 0 | 0 | 0 | 0 | 0 | 0 | 0 | 0 |
| Oligomeric galacturonic acid | wt.% | 0.00 | 0.02 | 0.12 | 0.04 | 0.01 | 000 | 0.00 | 0.00 | 0.00 | 0.00 | 0.00 |
| Citric acid | mg/kg | 0 | 0 | 0 | 0 | 0 | 0 | 0 | 0 | 0 | 0 | 0 |
| Malic acid | mg/kg | 0 | 0 | 0 | 0 | 0 | 0 | 0 | 0 | 0 | 0 | 0 |
| Lactic acid | mg/kg | 0 | 0 | 0 | 0 | 0 | 0 | 59 | 160 | 570 | 700 | 630 |
| Formic acid | mg/kg | 0 | 0 | 0 | 0 | 0 | 0 | 0 | 0 | 0 | 0 | 0 |
| Acetic acid | mg/kg | 0 | 0 | 170 | 0 | 0 | 52 | 110 | 72 | 200 | 300 | 280 |
| Pyrrolidone carboxylic acid (PCA) | mg/kg | 0 | 0 | 0 | 0 | 0 | 0 | 0 | 0 | 0 | 0 | 0 |
| Propionic acid | mg/kg | 0 | 0 | 0 | 0 | 0 | 0 | 0 | 0 | 0 | 0 | 0 |
| Butyric acid | mg/kg | 0 | 0 | 0 | 0 | 0 | 0 | 0 | 0 | 0 | 0 | 0 |

## Claims

1. Method of enriching a uronic acid containing composition, said method comprising the consecutive steps of:
a) providing a crude uronic acid composition comprising a first uronic acid and one or more monosaccharides;
b) subjecting the crude uronic acid composition of step a) to a chromatographic separation using a stationary phase comprising an anion exchange resin;
c) collecting an enriched uronic acid composition.

2. Method according to claim 1, wherein the first uronic acid is galacturonic acid.

3. Method according to claim 1 or 2 wherein the anion exchange resin used in step b) comprises tertiary or quaternary ammonium functional groups.

4. Method according to any one of the previous claims, wherein step b) is performed using an acidic mobile phase.

5. Method according to claim 4, wherein step b) is performed using a mobile phase comprising a mineral acid, preferably a mineral acid selected from the group consisting of sulfuric acid, hydrochloric acid, phosphoric acid, and nitric acid.

6. Method according to any one of the preceding claims, wherein the crude uronic acid composition comprises at least 1 wt.% of the first uronic acid based on dry matter of the composition, preferably at least 5 wt.%, more preferably at least 10 wt.%.

7. Method according to any one of the preceding claims, wherein the crude uronic acid composition comprises oligomeric first uronic acid.

8. Method according to claim 7, wherein the amount of oligomeric first uronic acid in the crude uronic acid composition is at least 0.05 wt.% of oligomeric uronic acid, based on dry matter, preferably at least 0.1 wt.%, preferably at least 0.3 wt.%, wherein the amount of oligomeric uronic first acid is determined according to the protocol of the detailed description.

9. Method according to any one of the preceding claims, wherein the total combined amount of fucose, rhamnose, arabinose, galactose, glucose, mannose, xylose, fructose, and sucrose in the crude uronic acid composition is at least 5 wt.%, based on dry matter of the composition, preferably at least 10 wt.%, more preferably at least 30 wt.%.

10. Method according to any one of the preceding claims, wherein the crude uronic acid composition comprises a biomass hydrolysate, such as a hydrolysate of pectin or of pectin-containing biomass such as a plant material hydrolysate, algae hydrolysate or bacteria hydrolysate.

11. Method according to any one of the previous claims wherein A_{crude}:B_{crude} < A_{enriched}:B_{enriched} wherein
A_{crude} is the concentration of first uronic acid, based on dry matter, in the crude uronic acid composition;
B_{crude} is the concentration of oligomeric first uronic acid, based on dry matter, in the crude uronic acid composition;
A_{enriched} is the concentration of first uronic acid, based on dry matter, in the enriched uronic acid composition;
B_{enriched} is the concentration of oligomeric first uronic acid, based on dry matter, in the enriched uronic acid composition;
wherein the amount of oligomeric first uronic acid is determined according to the protocol of the detailed description.

12. Method according to claim 11 wherein A_{ennched}:B_{ennched} is more than 20 times A_{crude}:B_{crude}.

13. Method according to any one of the previous claims wherein the chromatographic separation is performed by simulated moving bed chromatography.

14. Uronic acid containing composition, comprising:
- at least 50 wt.% of a first uronic acid, based on dry matter, preferably at least 70 wt.%, more preferably at least 90 wt.%;
- at least 0.1 wt.% of monosaccharides selected from arabinose and glucose, based on dry matter, preferably at least 0.25 wt.%;
- less than 0.1 wt.% of oligomeric uronic acid, based on dry matter, preferably less than 0.01 wt.%, more preferably less than 0.001 wt.%, wherein the amount of oligomeric uronic acid is determined according to the protocol of the detailed description.

15. Sugar raffinate composition comprising
- less than 4 wt.% of a uronic acid selected from the group consisting of galacturonic acid, mannuronic acid, guluronic acid, glucuronic acid, iduronic acid, and combinations thereof, based on dry matter, preferably less than 2 wt.%, more preferably less than 1 wt.%;
- at least 50 wt.% of monosaccharides selected from arabinose and glucose, based on dry matter, preferably at least 70 wt.%, more preferably at least 80 wt.%;
- more than 0.5 wt.% of the total combined amount of oligomeric uronic acids from uronic acids selected from the group consisting of galacturonic acid, mannuronic acid, guluronic acid, glucuronic acid, iduronic acid, and combinations thereof, based on dry matter, preferably more than 0.75 wt.%, more preferably more than 1 wt.%, wherein the amount of oligomeric uronic acid is determined according to the protocol of the detailed description; and
wherein preferably a total combined amount of uronic acids selected from the group consisting of galacturonic acid, mannuronic acid, guluronic acid, glucuronic acid, iduronic acid, and combinations thereof in the sugar raffinate composition is preferably less than 4 wt.%, based on dry matter.
